# EUROPÄISCHE PATENTSCHRIFT

(11) **EP 1 818 634 B1**
(45) Veröffentlichungstag und Bekanntmachung des Hinweises auf die Patenterteilung: **04.04.2018**
(21) Anmeldenummer: 07001403.0
(22) Anmeldetag: 23.01.2007
(51) Int. Cl.: F25J 3/02, C07C 7/00, C07C 11/04

(54) **Verfahren zur Kälteversorgung der Tieftemperaturtrennungsstufe einer Olefinanlage**
Method for providing refrigeration to the cryogenic separation stage of an olefin plant
Procédé pour fournir du froid à une étape de séparation cryogénique d'une installation de production d'oléfine

(30) Priorität: 08.02.2006 DE 102006005822
(43) Veröffentlichungstag der Anmeldung: 15.08.2007
(73) Patentinhaber: Linde AG, 80331 München (DE)
(72) Erfinder: Duc, Tuat Pham, 82377 Penzberg (DE)

(56) Entgegenhaltungen:
- CN-A- 1 407 304
- GB-A- 1 539 291
- US-A- 4 629 484
- US-A- 5 035 732
- US-A1- 2002 198 430
- US-A1- 2006 004 242

## Beschreibung

Die Erfindung betrifft ein Verfahren zur Tieftemperaturversorgung einer Tieftemperaturtrennungsstufe von Olefinen mit einem oder zwei Kohlenstoffatomen unter Verwendung eines Kältemittels in einer Anlage zur Olefinherstellung aus kohlenwasserstoffhaltigem Einsatz (Olefinanlage), wobei die Tieftemperaturtrennungsstufe drei Kondensationsstufen von -50°C bis -100°C umfasst und einer Fraktionierstufe nachgeschaltet ist, die Olefine mit höchstens zwei Kohlenstoffatomen von Olefinen mit mindestens drei Kohlenstoffatomen trennt (front end Deethanizer).

Olefine können zum Beispiel durch thermisches Spalten in einem Spaltofen (Steamcracker) aus längerkettigen Kohlenwasserstoffen hergestellt werden. Das nach dem Spaltprozess entstandene Gas (Rohgas) wird in mehreren Fraktionierstufen in Olefine mit einer unterschiedlichen Zahl von Kohlenstoffatomen getrennt. Nach dem Stand der Technik werden Olefine mit einem Kohlenstoffatom von Olefinen mit zwei Kohlenstoffatomen in einer Tieftemperaturtrennungsstufe separiert. Die Tieftemperaturtrennung erfolgt in drei Kondensationsstufen von -50°C bis -100°C, wobei nacheinander Ethylen, Ethan und Methan auskondensiert werden und gasförmiger Wasserstoff abgeleitet wird. Der Tieftemperaturtrennungsstufe ist in der Regel eine Fraktionierstufe vorgeschaltet, die Olefine mit höchstens zwei Kohlenstoffatomen von Olefinen mit mindestens drei Kohlenstoffatomen trennt (front end Deethanizer). Nach dem Stand der Technik wird die Kälteleistung für die drei Kondensationsstufen über den Temperaturbereich von -50°C bis -100°C durch einen Kältekreislauf erzeugt, bei dem verdampftes Ethylen als Kältemittel eingesetzt wird, welches durch entsprechende Verdichterkreisläufe inklusive Kompressor, Turbine, Wärmetauscher, Abscheider und Ablaufbehälter aus dem, von der Olefinanlage produzierten, Ethylengasstrom erzeugt wird. Der benötigte Verdichterkreislauf zur Ethylenkältemittelerzeugung erhöht dabei die Investitionskosten ebenso wie das Betriebsrisiko der Olefinanlage.

Die CN 1 407 304 A offenbart ein Verfahren zur Trennung eines leichten Kohlenwasserstoffgemischs. Bei dem Verfahren wird ein komprimiertes und gekühltes Kohlenwasserstoffrohgas in eine Gasphase und eine Flüssigkeit getrennt. Aus der Gasphase werden alle C3-Komponenten und die meisten der C2-Komponenten flüssig abgetrennt und einem Demethanizer zugeführt. Die verbleibende Gasphase wird gekühlt, geflasht und einem Wasserstoffsystem zugeführt.

Aus der US 2002/0198430 A1 ist ein Verfahren zur Rückgewinnung von Ethylen und schwereren Kohlenwasserstoffen einem durch Pyrolyse von Kohlenwasserstoffen erzeugten Gas offenbart, wobei flüssige Produkte aus einer fraktionierten Kondensation des Gases zur Rückgewinnung von Ethylen auf unterschiedlichen Höhen einem Deethanizer zugeführt werden.

Ein Verfahren und eine Anlage zur Rückgewinnung von Olefinen bei niedrigem Druck werden in der US 2006/0004242 A1 beschrieben. Ein Feedgas wird bei einem primären Destillationsdruck verdichtet destilliert. Ein Kopfstrom der Destillation wird bei niedrigem Druck gekühlt, teilweise kondensiert und als Rücklauf in der Destillation verwendet. US1539291 offenbart ein Verfahren entsprechend der Präambel von Anspruch 1. Der vorliegenden Erfindung liegt die Aufgabe zugrunde, ein Verfahren der eingangs beschriebenen Art so auszugestalten, dass die Kälteversorgung der Tieftemperaturtrennungsstufe der Olefinanlage auf wirtschaftliche Weise zuverlässig gewährleistet ist.

Diese Aufgabe wird durch ein Verfahren mit den Merkmalen des Patentanspruchs 1 gelöst. Die Aufgabe wird damit verfahrensseitig erfindungsgemäß dadurch gelöst, dass als Kältemittel ein Teil der flüssigen Olefinfraktion mit zwei Kohlenstoffen aus dem front end Deethanizer entnommen wird.

Die Investitionskosten für Verdichterkreisläufe zur Bereitstellung der Kälteleistung für die Tieftemperaturtrennungsstufe sind ein nicht unwesentlicher Teil der Investitionskosten einer Olefinanlage. Zusätzlich erhöht sich durch den Verdichterkreislauf das Risiko der Betriebsführung und stellt so ein Risiko für die Verfügbarkeit der Rohgastrennsequenz dar.

Der Grundgedanke der Erfindung besteht darin, auf den Verdichterkreislauf zur Ethylenkältemittelerzeugung zu verzichten und als Kältemittel einen Teil der flüssigen

Olefinfraktion mit zwei Kohlenstoffatomen aus dem front end Deethanizer zu verwenden.

Gemäß einer besonders bevorzugten Ausführungsform der Erfindung wird ein Teil der flüssigen Ethylenfraktion aus dem front end Deethanizer als Kältemittel für die drei Kondensationsstufen der Tieftemperaturtrennungsstufe verwendet. Vorteilhafterweise wird die entnommene, flüssige Ethylenfraktion über die Wärmetauscher der Kondensationsstufen geführt und dabei verdampft, wodurch die gewünschte Kälteleistung erzeugt wird. Das verdampfte Ethylen wird erfindungsgemäß zur Rohgasverdichtungsstufe der Olefinanlage zurückgeführt.

Eine Weiterbildung des Erfindungsgedankens sieht vor, dem front end Deethanizer und der Tieftemperaturtrennungsstufe eine katalytische Hydrierungsstufe zwischenzuschalten.

Mit der Erfindung gelingt es insbesondere, die notwendige Kälteleistung für die Tieftemperaturtrennungsstufe der Olefinanlage bereitzustellen. Gleichzeitig werden die Investitionskosten und die Risiken der Betriebsführung durch den Wegfall eines Verdichterkreislaufs inklusive Kompressor, Turbine, Wärmetauscher, Abscheider und Ablaufbehälter minimiert.

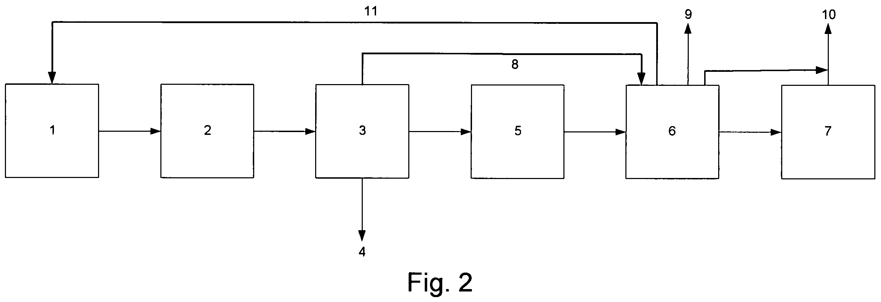

Im Folgenden soll die Erfindung anhand eines Vergleiches mit dem Stand der Technik mit dem in Figur 2 schematisch dargestellten Ausführungsbeispiel der Erfindung näher erläutert werden.

### Es zeigen

- Figur 1: ein Fließschema einer Olefinanlage mit front end Deethanizer, Tieftemperaturtrennungsstufe und Kälteversorgung nach dem Stand der Technik
- Figur 2: ein Fließschema einer Olefinanlage mit front end Deethanizer und anschließender katalytischer Hydrierungsstufe, Tieftemperaturtrennungsstufe und Kälteversorgung durch einen Teil der flüssigen Ethylenfraktion des Deethanizers

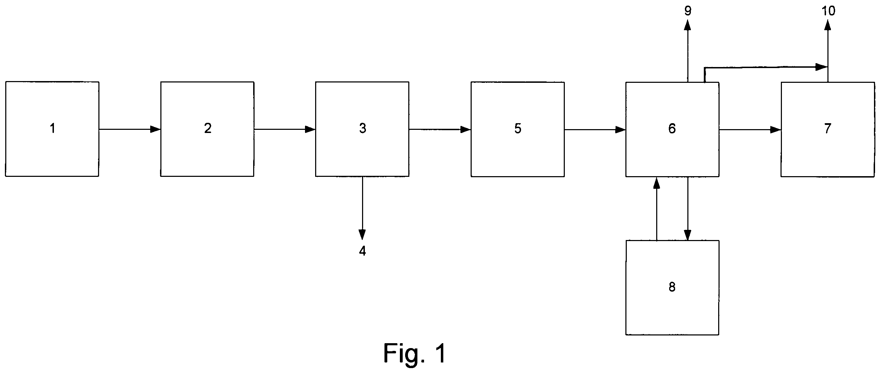

Das in Figur 1 dargestellte Fließschema zeigt einen Ausschnitt der Trennsequenz einer Olefinanlage nach dem Stand der Technik. Nach dem Rohgasverdichter (1), der Vorkühlung und Trocknung (2) wird das Rohgas in den front end Deethanizer (3) geführt, wo das Rohgas in eine Fraktion mit höchstens zwei Kohlenstoffatomen und eine Fraktion mit mindestens drei Kohlenstoffatomen getrennt wird, wobei die Fraktion mit mindestens drei Kohlenstoffatomen zur weiteren Trennsequenz der längerkettigen Olefine geführt wird (4). Die Olefinfraktion mit höchsten zwei Olefinen wird über eine katalytische Hydrierungsstufe (5) in die Tieftemperaturtrennungsstufe (6) geführt. Von der Tieftemperaturtrennungsstufe gelangen die Olefine zu einer weiteren Fraktionierungsstufe (7), in der Olefine mit einem Kohlenstoffatom von Olefinen mit zwei Kohlenstoffatomen getrennt werden. In der Tieftemperaturtrennungsstufe wird gasförmiger Wasserstoff (9) und Methan (10) abgeleitet. Die Kälteleistung für die drei Kondensationsstufen im Temperaturbereich von -50°C bis -100°C der Tieftemperaturtrennungsstufe (6) wird über einen separaten Verdichterkreislauf (8) bereitgestellt.

Figur 2 zeigt das Fließschema einer Ausgestaltung der Erfindung. Nach Rohgasverdichter (1), Vorkühlung und Trocknung (2) wird das Rohgas in den front end Deethanizer (3) geführt, wo das Rohgas in eine Fraktion mit höchstens zwei Kohlenstoffatomen und mindestens drei Kohlenstoffatomen getrennt wird. Die Fraktion mit mindestens drei Kohlenstoffatomen wird der weiteren Trennsequenz für längerkettige Olefine zugeführt (4), während die Fraktion mit höchstens zwei Kohlenstoffatomen über eine katalytische Hydrierungsstufe (5) zur Tieftemperaturtrennungsstufe (6) geführt wird, von wo aus das Rohgas zu einer Fraktionierungsstufe weitergeleitet wird, die Olefine mit einem Kohlenstoffatom von Olefinen mit zwei Kohlenstoffatomen trennt (7). Aus der Tieftemperaturtrennungsstufe werden gasförmiger Wasserstoff (9) und Methan (10) ausgeführt. Die Kälteleistung für die drei Kondensationsstufen der Tieftemperaturtrennungsstufe wird durch das Verdampfen einer flüssigen Ethylenfraktion (8) aus dem front end Deethanizer (3) über Wärmetauschern geliefert. Die verdampfte Ethylenfraktion (11) wird wieder zur Rohgasverdichtung (1) zurückgeführt.

## Patentansprüche

1. Verfahren zur Tieftemperaturversorgung in einer Anlage zur Herstellung von Olefinen aus kohlenwasserstoffhaltigem Einsatz unter Verwendung eines Kältemittels zur Tieftemperaturversorgung einer Tieftemperaturtrennungsstufe von Olefinen mit einem oder zwei Kohlenstoffatomen, wobei die Tieftemperaturtrennungsstufe (6) drei Kondensationsstufen von -50°C bis -100°C umfasst und einer Fraktionierstufe in Form eines front end Deethanizers (3) nachgeschaltet ist, die Olefine mit höchstens zwei Kohlenstoffatomen von Olefinen mit mindestens drei Kohlenstoffatomen (4) trennt **dadurch gekennzeichnet, dass** als Kältemittel eine flüssige Olefinfraktion mit zwei Kohlenstoffatomen (8) aus dem front end Deethanizer entnommen wird.

2. Verfahren nach Anspruch 1, **dadurch gekennzeichnet, dass** die flüssige Olefinfraktion mit zwei Kohlenstoffatomen über Gegenströmer verdampft wird.

3. Verfahren nach einem der Ansprüche 1 bis 2, **dadurch gekennzeichnet, dass** die in den Gegenströmern verdampfte Olefinfraktion mit zwei Kohlenstoffatomen zu einer Verdichtungsstufe der ungetrennten Olefine vor dem front end Deethanizer zurückgeführt wird.

4. Verfahren nach einem der Ansprüche 1 bis 3, **dadurch gekennzeichnet, dass** dem front end Deethanizer und der Tieftemperaturtrennungsstufe noch eine katalytische Hydrierungsstufe zwischengeschaltet ist.

## Claims

1. Method for the low-temperature supply in a plant for producing olefins from hydrocarbon-containing feed using a refrigerant, for the low-temperature supply of a low-temperature separation stage of olefins having one or two carbon atoms, with the low-temperature separation stage (6) comprising three condensation stages from -50°C to -100°C and being connected downstream of a fractionation stage in the form of a front end deethanizer which separates olefins having at most two carbon atoms from olefins having at least three carbon atoms (4), **characterized in that**, as refrigerant, a liquid olefin fraction having two carbon atoms (8) is taken off from the front end deethanizer.

2. Method according to Claim 1, **characterized in that** the liquid olefin fraction having two carbon atoms is vaporized via countercurrent flow heat exchangers.

3. Method according to one of Claims 1 to 2, **characterized in that** the olefin fraction having two carbon atoms which is vaporized in the countercurrent flow heat exchangers is recirculated to a compressor stage of the unseparated olefins upstream of the front end deethanizer.

4. Method according to one of Claims 1 to 3, **characterized in that** a catalytic hydrogenation stage is further connected between the front end deethanizer and the low-temperature separation stage.

## Revendications

1. Procédé d'alimentation à basse température dans une unité pour la fabrication d'oléfines à partir d'une charge contenant des hydrocarbures en utilisant un agent réfrigérant pour l'alimentation à basse température d'une étape de séparation à basse température d'oléfines contenant un ou deux atomes de carbone, l'étape de séparation à basse température (6) comprenant trois étapes de condensation de -50 °C à -100 °C et étant raccordée en aval d'une étape de fractionnement sous la forme d'un dé-éthaniseur initial (3), qui sépare des oléfines contenant au plus deux atomes de carbone d'oléfines contenant au moins trois atomes de carbone (4), **caractérisé en ce qu'**une fraction liquide d'oléfines contenant deux atomes de carbone (8) est extraite du dé-éthaniseur initial en tant qu'agent réfrigérant.

2. Procédé selon la revendication 1, **caractérisé en ce que** la fraction liquide d'oléfines contenant deux atomes de carbone est évaporée par des appareils à contre-courant.

3. Procédé selon l'une quelconque des revendications 1 à 2, **caractérisé en ce que** la fraction d'oléfines contenant deux atomes de carbone évaporée dans les appareils à contre-courant est recyclée dans une étape de compression des oléfines non séparées avant le dé-éthaniseur initial.

4. Procédé selon l'une quelconque des revendications 1 à 3, **caractérisé en ce qu'**une étape d'hydrogénation catalytique est encore raccordée entre le dé-éthaniseur initial et l'étape de séparation à basse température.
